# EUROPEAN PATENT APPLICATION

(11) **EP 1 496 057 A1**
(43) Date of publication of application: **12.01.2005**
(21) Application number: 03725594.0
(22) Date of filing: 16.04.2003
(51) Int. Cl.: C07D 403/04, A61K 31/498, A61P 9/00, A61P 9/10, A61P 25/00, A61P 25/08, A61P 25/14, A61P 25/16, A61P 25/18, A61P 43/00

(54) **NOVEL CRYSTAL OF QUINOXALINEDIONE DERIVATIVE ANHYDRIDE**

(30) Priority: 17.04.2002 JP 2002114781
(71) Applicant: YAMANOUCHI PHARMACEUTICAL CO. LTD., Tokyo 103-8411 (JP)
(72) Inventor: YUDA, Masamichi, Tsukuba-shi, Ibaraki 305-8585 (JP); KOHINATA, Takeru, Yamanouchi Pharma. Co., Ltd., Tokyo 174-8612 (JP)
(74) Representative: Bates, Philip Ian
(86) International application number: PCT/JP2003/004844
(87) International publication number: WO 2003/087091

(57) **Abstract**

The object of the present invention is to provide a novel crystal of a quinoxalinedione derivative as an AMPA antagonist, which enables providing a bulk for manufacturing pharmaceuticals that are stable under any humidity conditions.

## Description

### Technical Field

The present invention relates to a novel α crystal (hereinafter, referred to as a compound (I)) of free form anhydride of [7-(1H-imidazol-1-yl)-6-nitro-2,3-dioxo-3,4-dihydroquinoxalin-1(2H)-yl]acetic acid (hereinafter, referred to as compound A) which has excellent stability against humidity, and to an AMPA receptor antagonist which contains the compound (I) as an active ingredient, especially a therapeutic agent for treating cerebral infarction.

### Background of the Invention

It has been reported that a tautomer, a salt, a hydrate or a solvate of [7-(1H-imidazol-1-yl)-6-nitro-2,3-dioxo-3,4-dihydroquinoxalin-1(2H)-yl]acetic acid (compound A) has high affinity for the AMPA receptor and exhibits potent activities in the inhibitory action against kainic acid neurotoxicity and an anticonvulsant effect for audiogenic seizure in DBA/2 mouse, and based on these actions, is a particularly useful psychotropic agent for the prevention and treatment of Huntington's chorea, Parkinson's disease, epilepsy, Alzheimer's disease or senile dementia, and also as an anti-ischemia agent for the prevention and treatment of cell death caused by cerebral ischemia, oxygen deficiency or temporary cardiac arrest, neurodegeneration observed after hypoglycemia and seizure, and deficiency of mental and motor function, and has excellent solubility (Patent Reference 1).

Patent Reference 1 discloses that a series of tetrahydroquinoxalinedione derivatives generally can form crystal polymorphism [lines 16 to 19, right column of page (5) in Patent Reference 1], that a crystal of the compound A ·1 hydrochloride · 1.2 hydrate is obtained and the elemental analysis indicates that the crystal has 1.2 mole of water in Example 1 [line 21 from the bottom thereof, right column of page (9) to line 5, right column of page (10), especially lines 39 to 50, left column of page (10)], that a crystal of the compound A ·1 hydrochloride · 1 hydrate is obtained in Example 23 [line 27, right column of page (18) to line 25, right column of page (19), especially lines 16 to 25, left column of page (19)], and that a crystal of the compound A is obtained and the elemental analysis indicates that the crystal has 0.2 mole of water in Example 24 [lines 26 to 41, especially lines 30 to 41, left column of page (19)], respectively.

On the other hand, Patent Reference 2 discloses an improved method of producing 1 hydrate of free form of the compound A in the examples.

Further, it has been reported that 1 hydrate of the compound A has high water-solubility, and has excellent reducing effect on the volume of cerebral infarction in focal cerebral ischemia model (Non-Patent Reference 1 and Non-Patent Reference 2).

However, it has never been disclosed in the above-mentioned Patent References and Non-Patent References that an anhydride of the free form, and further crystal polymorphism thereof exist concretely, although Example 24 of Patent Reference 1 has implicated the possibility of a mixture of an anhydride of the free form compound A and a hydrate thereof.

In addition, data implicating the existence of α crystal of the present invention have not been provided in the analysis data of Example 24 of Patent Reference 1.
Patent Reference 1: Japanese Patent No. 2865878
Patent Reference 2: JP-A-2000-281676
Non-Patent Reference 1: Society for Neuroscience Abstracts 22, Part 2, 604.2, 1996
Non-Patent Reference 2: The Japanese Journal of Pharmacology 76 Suppl. I, P-362, P-369, P-372, 1998

### Problems to be resolved by the Invention

Under such technologies, the present inventors have investigated the compound A, and found that the free form compound A was usually obtained as 1 hydrate according to the production method of Patent Reference 2, and such 1 hydrate should be refrigerated to ensure stability since the 1 hydrate may increase impurities depending on storage condition, therefore, it is not industrially sufficient as a bulk for manufacturing pharmaceuticals.

Therefore, an object of the present invention is to provide a stable crystal of the compound A which can be used as a bulk for manufacturing pharmaceuticals of the compound A which has excellent pharmacological activity and pharmaceutical properties.

### Means for solving the Problems

Under such circumstances, the present inventors have conducted extensive studies on a stable crystal which can be provided as a bulk of pharmaceuticals of the compound A, and have found that free forms of the compound A form an anhydride thereof, there are crystal polymorphism consisting of two types in the anhydride of the free forms, i.e., α-form (compound (I)) and β-form (compound (II)), and that the P crystal is unstable against humidity so that it is not suitable to be provided as a bulk of pharmaceuticals. The present inventors have further investigated regarding a crystal which is stable against humidity, and as a result, found that the above-mentioned compound (I) has excellent stability against humidity, and have completed the present invention.

That is, the present invention provides an α crystal of free form anhydride of [7-(1H-imidazol-1-yl)-6-nitro-2,3-dioxo-3,4-dihydroquinoxalin-1(2H)-yl]acetic acid (compound A).

Preferably, the α crystal exhibits peaks at diffraction angles of 9.1°, 19.4°, 22.5°, 23.3°, 23.9°, 25.7° and 26.2° in X-ray powder diffraction patterns.

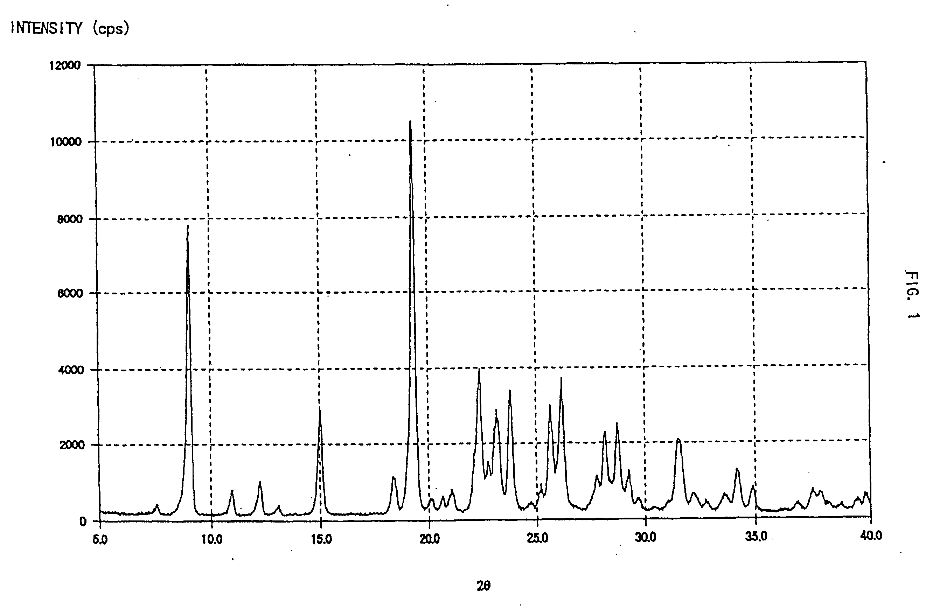

More preferably, the α crystal exhibits diffraction peaks at the same diffraction angles as those of the X-ray powder diffraction pattern shown in Fig. 1.

Most preferably, the α crystal exhibits peaks at the same diffraction angles as those of the X-ray powder diffraction pattern shown in Fig. 1 and further exhibits an exothermic peak accompanying decomposition in the vicinity of 341°C according to thermal analysis (TG-DSC).

According to another aspect of the present invention, the present invention relates to a pharmaceutical as an AMPA receptor antagonist which comprises the above-mentioned α crystal of free form anhydride of the compound A as an active ingredient, preferably a therapeutic agent for treating cerebral infarction.

Further, the present invention relates to the use of the α crystal for manufacturing pharmaceuticals for treating cerebral infarction, comprising a therapeutically effective amount of the above-mentioned α crystal of the free form anhydride of the compound A.

Furthermore, the present invention relates to a method of treating cerebral infarction comprising administering to a patient a therapeutically effective amount of the above-mentioned α crystal of the free form anhydride of the compound A.

### Brief Description of the Drawings

Fig. 1 shows an X-ray powder diffraction pattern of the compound (I);
Fig. 2 shows TG-DSC curves of the compound (I);
Fig. 3 shows an X-ray powder diffraction pattern of the compound (II); and
Fig. 4 shows TG-DSC curves of the compound (II).

### Description of the Preferred Embodiments

The present invention will be now explained in detail below.

The compound (I) of the present invention, i.e., α crystal of free form anhydride of [7-(1H-imidazol-1-yl)-6-nitro-2,3-dioxo-3,4-dihydroquinoxalin-1(2H)-yl]acetic acid is preferably a compound which exhibits peaks at diffraction angles of 9.1°, 19.4°, 22.5°, 23.3°, 23.9°, 25.7° and 26.2°in the X-ray powder diffraction patterns measured using Cu-Kα rays.

In addition, the diffraction angles usually mean ones which coincide in the range of 2θ ± 0.2°. The peaks mean main peaks and include peaks not larger than medium at diffraction angles other than those stated above.

More preferably, the compound (I) of the present invention is a compound which exhibits peaks at the same diffraction angles as those of the X-ray powder diffraction pattern shown in Fig. 1.

Even more preferably, the compound (I) of the present invention is a compound which exhibits peaks at the same diffraction angles as those of the X-ray powder diffraction pattern shown in Fig. 1, and has same the relative intensity.

Most preferably, the compound (I) of the present invention is a compound which exhibits peaks at the same diffraction angles as those of the X-ray powder diffraction pattern shown in Fig. 1 and further exhibits an exothermic peak accompanying decomposition in the vicinity of 341°C according to thermal analysis (TG-DSC).

As used herein, X-ray diffractometry is preferably carried out using Cu-Kα rays. However, crystal lattice spacing and an overall pattern is critical for identifying the crystal in view of data characteristics, and the relative intensity should not be strictly interpreted since it may be varied depending on the direction of crystal growth, particle sizes and measurement conditions.

The compound (I) of the present invention can be prepared by adding 1 hydrate of the compound A to warm water, stirring it under light-shielding condition, and filtering and drying the obtained crystal.

1 hydrate of the compound A used as a raw material can be prepared by the production methods described in the above references, or by the production methods described in Patent Reference 2.

The compound (I) of the present invention is useful as a therapeutic agent for treating cerebral infarction, preferably acute cerebral infarction.

The treatment of cerebral infarction herein refers to effects of inhibiting the progress of cerebral infarct during the acute period of cerebral infarction, effects of improving the dysfunction or subjective symptoms associated with cerebral infarction, and/or effects of suppressing occurrence of mental symptoms or convulsive seizure during chronic period of cerebral infarction. Further, the treatment of cerebral infarction herein includes the prevention of recurrent attacks of cerebral infarction.

The acute period of cerebral infarction herein means within 48 hours from the onset.

The degree of cerebral infarction can be classified by CT findings before administration according to infarct size, infarct breadth (perforating arteries, cortical branches), infarct side (left, right, both sides), infarct area (anterior cerebral artery area, middle cerebral artery area, posterior cerebral artery area, watershed area, brainstem, cerebellum, and the like) and extent of edema.

The dysfunction associated with cerebral infarction herein means neurological symptoms occurring during the acute period of cerebral infarction, disorders in performing daily activities, motor paralysis, and the like.

Such symptoms can be represented by index of neural function and disorders using neurological check (assessment of consciousness level, pupil symmetry and light response, symptoms such as ataxia and tonic muscular contraction), modified Rankin Scale, Barthel Index, NIHSS, and Glasgow Scale.

Specifically, the following clinical symptoms are observed and quantified.

### Consciousness disorders

Subjective symptoms (headache, heavy headedness, dizziness, numbness of limbs and the like)
Mental signs (reaction to calling or greeting, orientation, volition, knowledge, calculation ability, voice tone, attitude, voluntary movement, voluntary speech, attention, and the like)
Neurological signs (aphasia, apraxia, agnosia, dysarthria, dysphagia, muscular strength on the side of motor paralysis, sensory disorders, and the like)
Disorders in performing daily activities (turning body in bed, maintenance of sitting position, standing up, walking, dressing and undressing, feeding, miction and bowel movement, and the like)

The neurological symptoms refer to a state having one or more disorders listed above for the neurological signs.

The chronic mental symptoms mean mental symptoms that are occurred within 3 weeks after the onset of cerebral infarction, specifically, declined voluntariness (hypobulia), memory disorders, anxiety, depression, bad mood, nervousness, delusion, emotional sensitiveness, aggressive behavior, mental excitement, wandering, delirium, and the like.

The compound (I) of the present invention can be administered by various routes including parentally, specifically, subcutaneously, intramuscularly, intravenously, percutaneously, intra-marrow, epidurally, intraarticularly, and topically, or if possible, orally.

Examples of formulations for parenteral administration include sterile aqueous or non-aqueous solution, suspension and emulsion. The aqueous solution or suspension includes, for example, distilled water for injection and physiological saline. Examples of the non-aqueous solution or suspension include propylene glycol, polyethylene glycol, vegetable oil such as olive oil, alcohols such as ethanol, "Polysorbate 80" (trade name) and the like. Such compositions may further contain an additive such as antiseptics, a wetting agent, an emulsifying agent, a dispersing agent, a stabilizing agent (e.g., lactose), or an aid such as a solubilizing agent (e.g., megluminic acid). They are sterilized, for example, by filtration through a bacteria-retaining filter, incorporation of a bactericide, or irradiation. They can also be obtained by first preparing a sterile solid composition and then dissolving it in a sterile water or sterile solvent for injection before use. The compositions are preferably lyophilized formulations.

The dose of the compound (I) according to the present invention is determined appropriately according to each case in consideration of the symptoms, age or sex of the subject to be administered, and the like. The general dose ranges from 100 to 2000 mg/day, preferably about 900 mg/day, per adult. 100 to 2000 mg/day per adult is administered in a single dose or in divided doses of two to four. In the case of intravenous administration or in the case of continuous intravenous administration, it may be administered for 1 hour/day to 24 hours/day.

As described above, the dose may vary depending on various conditions. If effective, the dose smaller than the above range may be sufficient.

### Examples

In the following, the present invention will be explained in detail by way of Examples, but the present invention will not be limited thereto.

### Example 1:

### 1) Preparation of anhydride of the compound A

About 10 g of 1 hydrate of the compound A was added to 500 ml of warm water of about 50°C, and the solution was stirred (200 rpm) for 2 days with a paddle under light-shielding condition. After suction filtration, the residue was dried at 80°C for 3 days under reduced pressure to give dark-yellow crystalline powder (compound (I)). In addition, 1 hydrate of the compound A was dried at 105°C for 1 hour to give light-yellow crystalline powder (compound (II)).

### 2) Identifying the crystalline form

Measurements of X-ray powder diffraction and thermal analysis were carried out for the products obtained in 1). In addition, the compound (II) was stored in a silica gel desiccator just until the measurements, and sampling for measurement was also carried out under the condition as dry as possible.

As a result, the products were identified as α form of anhydride crystal (compound (I)) and β form of anhydride crystal (compound (II)).

### (1) α form of anhydride crystal (compound (I))

The α form of anhydride crystal (compound (I)) is obtained as dark-yellow crystalline powder and, as shown above, is a compound which exhibits peaks in the vicinity of diffraction angles of 9.1°, 19.4°, 22.5°, 23.3°, 23.9°, 25.7° and 26.2°in the X-ray powder diffraction patterns measured using Cu-Kα rays.

Further, the compound (I) of the present invention exhibits an exothermic peak accompanying decomposition in the vicinity of 341°C according to thermal analysis (TG-DSC).

The X-ray powder diffraction pattern of the compound (I) is shown in Fig. 1.

Measurement conditions: equipment; X-ray powder diffractometer (MXP 18TAHF 22, available from MAC Science K.K.), anticathode: Cu, tube voltage: 40 KV, tube current; 40 mA, scanning speed: 3.0°/min.

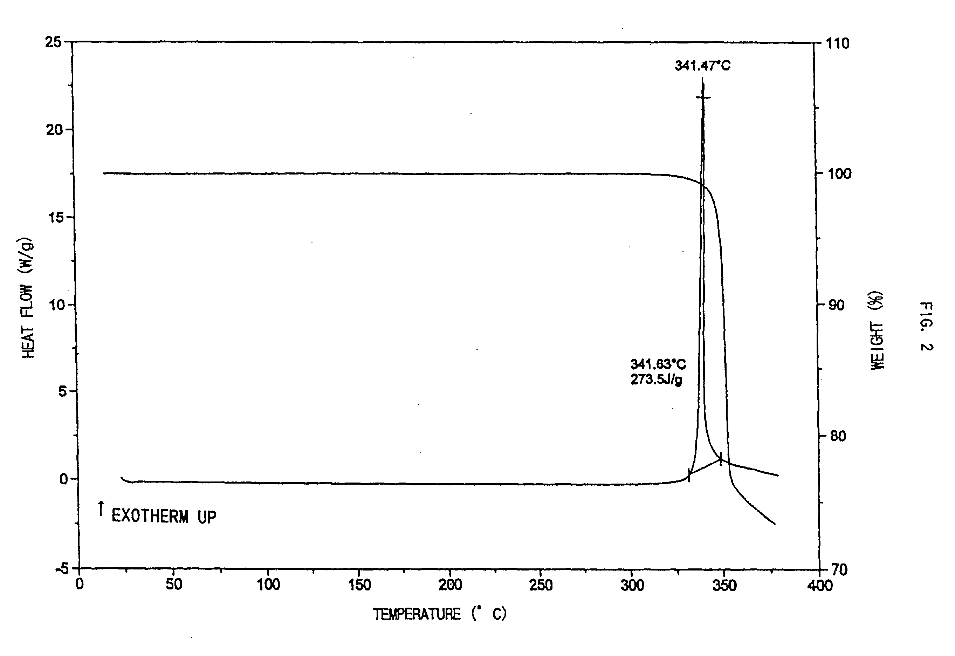

In addition, the results from TG-DSC thermal analysis are shown in Fig. 2.

### Measurement conditions:

TG equipment; thermogravimetric analyzer (TA Instrument 2950), sample amount; about 8 mg, kind of atmospheric gas; nitrogen, total flow rate of the atmospheric gas; 100 ml/min, heating rate; 10°C/min.

DSC equipment; differential scanning calorimeter (TA Instrument 2910), sample amount; about 5 mg, reference substance; empty pan, sample pan; aluminum open pan, kind of atmospheric gas; nitrogen, flow rate of the atmospheric gas; 50 ml/min, heating rate; 10°C/min.

### (2) β form of anhydride crystal (compound (II))

The β form of anhydride crystal (compound (II)) is obtained as light-yellow crystalline powder and a compound which exhibits peaks in the vicinity of diffraction angles of 10.5°, 18.8°, 21.2°, 23.5°, 26.7° and 29.5° in the X-ray powder diffraction patterns measured using Cu-Kα rays.

Further, the compound (II) of the present invention exhibits an exothermic peak accompanying decomposition in the vicinity of 320°C according to thermal analysis (TG-DSC).

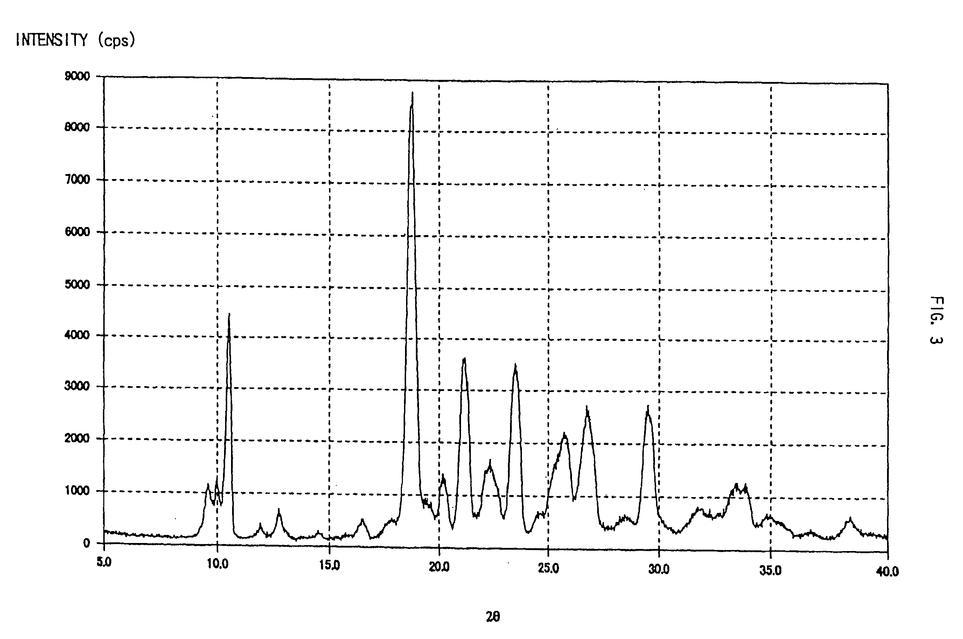

The X-ray powder diffraction pattern of the compound (II) is shown in Fig. 3.

Measurement conditions: equipment; X-ray powder diffractometer (MXP 18TAHF 22, available from MAC Science K.K.), anticathode: Cu, tube voltage: 40 KV, tube current; 40mA, scanning speed: 3.0°/min

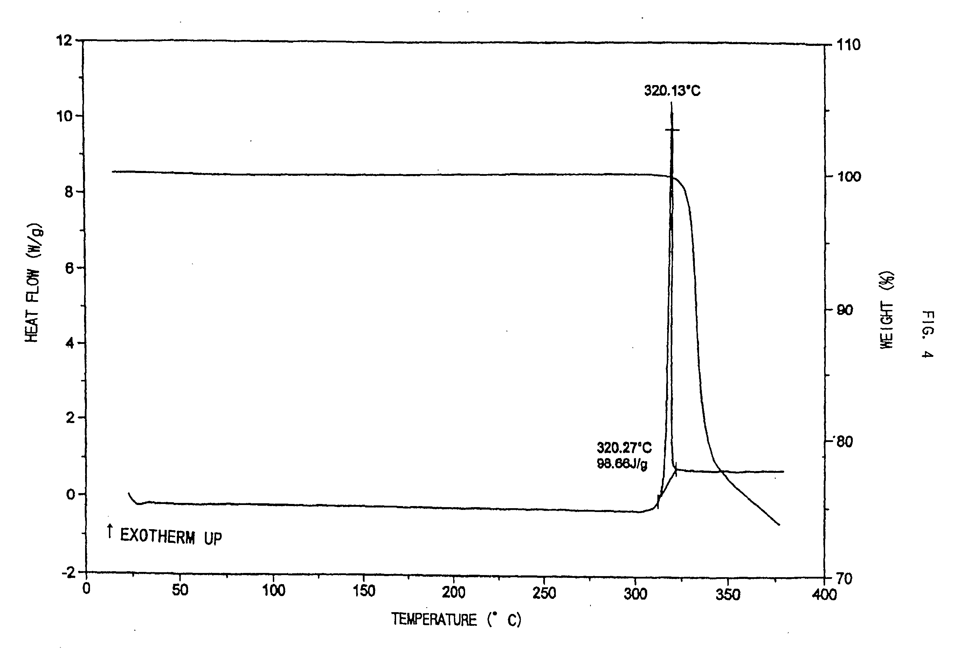

In addition, the results from TG-DSC thermogravimetric analysis are shown in Fig. 4.

### Measurement conditions:

TG equipment; thermogravimetric analyzer (TA Instrument 2950), sample amount; about 7 mg, kind of atmospheric gas; nitrogen (TA Instrument 2950), total flow rate of the atmospheric gas; 100 ml/min, heating rate; 10°C/min.

DSC equipment; differential scanning calorimeter (TA Instrument 2910), sample amount; about 6 mg, reference substance; empty pan, sample pan; aluminum open pan, kind of atmospheric gas; nitrogen, flow rate of the atmospheric gas; 50 ml/min, heating rate; 10°C/min.

### 3) Hygroscopicity test

About 1 g each of the compound (I) and the compound (II) of anhydride compound A were weighed accurately in weighing bottles (seven) of known weight, then stored under the following conditions at 25°C, and time course weight changes were measured.

RH herein means relative humidity.

**Table 1**

| Salt-saturated solution | %RH |
|---|---|
| (1) Magnesium chloride MgCl₂ · 6H₂O | 33 |
| (2) Calcium nitrate Ca(NO₃)₂ · 4H20 | 51 |
| (3) Ammonium nitrate NH₄NO₃ | 63.5 |
| (4) Sodium chloride NaCl | 75 |
| (5) Potassium chloride KCl | 84 |
| (6) Potassium nitrate KNO₃ | 93 |
| Drying agent | %RH |
| (7) Silica gel | ∼0%RH |

The results are shown in Table 2-1 and Table 2-2.

**Table 2-1:**

| Time course weight changes of the compound (I) | | | | |
|---|---|---|---|---|
| RH | Day | | | |
| | 1 | 2 | 4 | 7 |
| ∼0% | 0.03 | -0.01 | 0.02 | 0.01 |
| 33% | 0.09 | 0.06 | 0.10 | 0.08 |
| 51% | 0.03 | 0.04 | 0.10 | 0.09 |
| 63.5% | 0.03 | 0.06 | 0.10 | 0.11 |
| 75% | 0.04 | 0.07 | 0.13 | 0.10 |
| 84% | 0.05 | 0.09 | 0.11 | 0.13 |
| 93% | 0.09 | 0.10 | 0.15 | 0.13 |

**Table 2-2:**

| Time course weight changes of the compound (II) | | | | |
|---|---|---|---|---|
| RH | Day | | | |
| | 1 | 2 | 4 | 7 |
| ∼0% | 0.09 | 0.08 | 0.07 | 0.04 |
| 33% | 5.26 | 5.29 | 5.32 | 5.33 |
| 51% | 5.39 | 5.39 | 5.40 | 5.38 |
| 63.5% | 5.47 | 5.47 | 5.49 | 5.49 |
| 75% | 5.47 | 5.47 | 5.51 | 5.46 |
| 84% | 5.51 | 5.52 | 5.52 | 5.51 |
| 93% | 5.52 | 5.49 | 5.54 | 5.51 |

From the above results, the compound (I) was stable under any relative humidity conditions almost without showing hygroscopicity (Table 2-1). On the other hand, the compound (II) showed strong hygroscopicity, and absorbed water corresponding to 1 mole after one day under the conditions of at least 33% RH or more (Table 2-2).

Accordingly, the compound (I), which is stable under any humidity conditions from low humidity to high humidity, and therefore is useful as a bulk for manufacturing pharmaceuticals without requiring special containers or storage conditions for controlling the temperature and humidity.

### Example 2: (Preparation of lyophilized formulation)

33.3 g of meglumine was dissolved in 400 ml of water for injection, and to this solution was added 10 g of the compound (I) with stirring to dissolve the latter. To this solution was added 1400 ml of water for injection, 40 g of mannitol was dissolved therein, and thereto was added water for injection to make the total volume of 2000 ml of the solution. This solution was subjected to sterile filtration by a conventional method, and 15 ml of the filtered solution was filled into a vial of a volume of 30 ml, followed by lyophilization by a conventional method to give a lyophilized formulation of the compound (I) that is used in the present invention.

### Experimental Example:

### Pharmacological effects of the compound according to the present invention can be evaluated as follows:

### 1) Measurement of inhibitory activity against the binding of [³H]-AMPA:

In ice water, a reaction solution containing 0.5 ml in total of a reaction solution containing about 45 nM [³H]-AMPA [2-amino-3-(3-hydroxy-5-methyl-4-isoxazole)propionic acid], about 300 mg of a sample of a rat cerebral membrane and a test compound was reacted for 45 minutes. The amount of [³H]-AMPA bound to the AMPA receptor was measured by a filtration method. A portion substituted by 10 µM quisqualic acid in the total bound amount was taken as the specific binding amount. The test compound was evaluated by determining the binding inhibition ratio against the specific binding.

### 2) Measurement of inhibitory activity against kainic acid neurotoxicity:

Inhibitory action of the compound of this invention against kainic acid neurotoxicity was investigated using the primary culture system of the hippocampal neuron of a fetal rat.

### (1) Incubation conditions

The hippocamp was cut out from the brain of an 18 to 20-day-old fetal rat and was subjected to enzyme treatment with papain and DNase I to disperse the cells. The cells so obtained were floated on MEM containing 10% serum and then were inoculated on a 48-well plate treated in advance with poly-1-lysine in a concentration of 4 × 10⁵ cell/cm². Twenty four hours later, the serum was replaced by a serum-free medium. The medium replacement was carried out twice a week. The cells which were cultivated for at least 6 days were provided to the following test.

### (2) Inhibition against kainic acid neurotoxicity

Neurotoxicity was expressed by an activity of lactate dehydrogenase released in the culture medium by the cell death. As a control, neurons which were exposed to a serum-free medium containing 300 µM kainic acid for 24 hours were used. Each compound, together with 300 µM kainic acid, was allowed to act on the neuron for 24 hours and inhibitory action of each compound against neuronal death caused by kainic acid was evaluated.

### 3) Inhibitory action on cerebral infarction (MCA (middle cerebral artery) Permanent Occlusion Model)

In accordance with the known report (J. Pharmacol. Exp. Thr., 276, 84-92, 1996), the left-side MCA of a Fischer-344 rat was permanently occluded, and 24 hours of continuous intravenous infusion of the compound of the invention was started 5 minutes thereafter without anesthesia and immobilization. After completion of the administration, the rat was decapitated to excise the brain which was subsequently stained with 2,3,5-triphenyltetrazolium hydrochloride (TTC) to measure the infarction volume.

### Effect of the Invention

According to the present invention, the compound (I) is provided, which is a novel physicochemically stable crystal form. The compound (I) of the present invention is stable under any conditions from low humidity to high humidity, and therefore useful as a bulk for manufacturing pharmaceuticals.

## Claims

1. An α crystal of free form anhydride of [7-(1H-imidazol-1-yl)-6-nitro-2,3-dioxo-3,4-dihydroquinoxalin-1(2H)-yl]acetic acid.

2. The α crystal according to claim 1, wherein the crystal exhibits peaks at diffraction angles of 9.1°, 19.4°, 22.5°, 23.3°, 23.9°, 25.7° and 26.2° in X-ray powder diffraction patterns.

3. The α crystal according to claim 2, wherein the crystal exhibits diffraction peaks at the same diffraction angles as those of the X-ray powder diffraction pattern shown in Fig. 1.

4. The α crystal according to claim 3, wherein the crystal exhibits an exothermic peak accompanying decomposition in the vicinity of 341°C according to thermal analysis (TG-DSC).

5. An AMPA receptor antagonist comprising the α crystal of free form anhydride of the compound A according to any one of claims 1 to 3, as an active ingredient.

6. A pharmaceutical comprising the crystal according to claim 3, as a therapeutic agent for treating cerebral infarction.

7. Use of the α crystal for manufacturing a pharmaceutical for treating cerebral infarction, comprising a therapeutically effective amount of the α crystal according to any one of claims 1 to 3.

8. A method of treating cerebral infarction, comprising administering to a patient a therapeutically effective amount of the α crystal according to any one of claims 1 to 3.
